(19) Europäisches Patentamt
European Patent Office
Office européen des brevets

(11) Veröffentlichungsnummer : **0 061 687**
**B1**

(12) # EUROPÄISCHE PATENTSCHRIFT

(45) Veröffentlichungstag der Patentschrift : **24.04.85**

(51) Int. Cl.⁴ : **C 12 P 7/40**, C 12 P 17/06// C07C59/11, C07D311/94

(21) Anmeldenummer : **82102355.3**

(22) Anmeldetag : **22.03.82**

(54) **Verfahren zur Herstellung eines tricyclischen Steroid-Abbauproduktes.**

(30) Priorität : **01.04.81 DE 3113053**

(43) Veröffentlichungstag der Anmeldung : **06.10.82 Patentblatt 82/40**

(45) Bekanntmachung des Hinweises auf die Patenterteilung : **24.04.85 Patentblatt 85/17**

(84) Benannte Vertragsstaaten : **AT BE CH DE FR GB IT LI LU NL SE**

(56) Entgegenhaltungen :
**FR-A- 2 387 288**
**US-A- 4 062 729**
**JOURNAL OF THE CHEMICAL SOCIETY, Perkin Transactions I, April 1978, Seiten 336-340, London, G.B. U. SCHOMER et al.: "Microbial transformation of beta-sitosterol: Accumulation of 3-(5alpha-hydroxy-7abeta-methyl-1-oxo-3aalphaH-hexahydroindan-4alpha-yl)porpionic. Acid and the X-ray structural identification of its gamma-lactone"**
**AGRICULTURAL AND BIOLOGICAL CHEMISTRY, Band 44, Nr. 7, Juli 1980, Seiten 1469-1474, Tokyo, JP. TSUYOSHI NAKAMATSU et al.: "Microbial degradation of steroids to hexahydroindanone derivates"**

(73) Patentinhaber : **Henkel Kommanditgesellschaft auf Aktien**
**Postfach 1100 Henkelstrasse 67**
**D-4000 Düsseldorf-Holthausen (DE)**

(72) Erfinder : **Schindler, Joachim, Dr.**
**Am Eichelkamp 158**
**D-4010 Hilden (DE)**
Erfinder : **Schmid, Rolf, Dr.**
**Am Nettchesfeld 30**
**D-4000 Düsseldorf 13 (DE)**

**EP 0 061 687 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Es ist bekannt, daß gewisse Mikroorganismen, insbesondere bestimmte Mikroorganismen-Defektmutanten als intermediäres Abbauprodukt von Steroid-Verbindungen Hexahydro-indanon-Derivate bilden. So ist es beispielsweise bekannt, daß ein von Nocardia corallina IFO 3338 abgeleiteter Mutantenstamm aus Cholesterin unter anderem 3a α-H-4α-[3'-Propionsäure]-5α-hydroxy-7aβ-methylhexahydro-1-indanon-δ-Lacton der Strukturformel

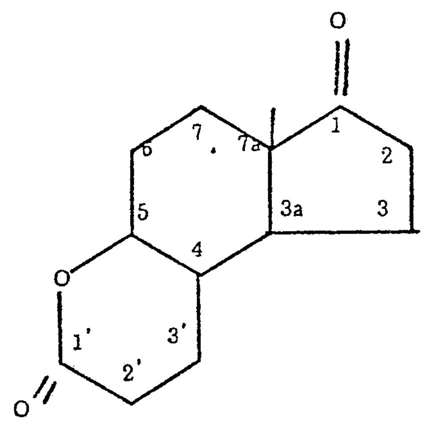

(I)

bildet — siehe T. Nakamatsu Agric. Biol. Chem. 44 (7), 1469-1474 (1980). Als primäres Reaktionsprodukt der Fermentationsstufe entsteht dabei die zugehörige freie Hydroxycarbonsäure 3-(5α-Hydroxy-7aβ-methyl-1-oxo-3aα-H-hexa-hydroindan-4α-yl)-propionsäure. Durch Ansäuern der dieses Abbauprodukt enthaltenden Kulturlösung, beispielsweise auf einen pH-Wert von 2,0 oder darunter mit Schwefelsäure, bildet sich das zuvor angegebene δ-Lacton — siehe hierzu FR PS 2 387 288. Andere Autoren berichten über die Umwandlung von Sitosterin mit der Mutante Nocardia sp. M 29 zur α-Hydroxycarbonsäure der angegebenen Struktur — vergleiche U. Schömer et. al. European J. Appl. Microbiol. Biotechnol. 10,99-106 (1980). Weitere Hinweise auf die Bildung dieses partiellen Abbauproduktes von Sterinen, insbesondere Sitosterol, finden sich in den US- PSen 4.176.123 und 4.042.459 sowie in der DE-OS 2 746 323. Auch für den mikrobiellen Abbau von Gallensäuren ist die Bildung des Hexahydro-1-indanon-δ-Lactons nachgewiesen worden — vergleiche hierzu beispielsweise Nakamatsu et. al. aaO, Seite 1473 und S. Hashimoto et. al. Biochem. J. 164,715 (1977).

Gegenstand der vorliegenden Erfindung ist die Verwendung einer bisher nicht beschriebenen Mutante K 14, die durch UV-Behandlung aus dem cholesterinabbauenden Wildstamm Chol 149 isoliert wurde, zum mikrobiellen Abbau von Steroid-Verbindungen tierischen und pflanzlichen Ursprungs in einem wäßrigen Nährmedium unter aeroben Bedingungen zur 3-(5α-Hydroxy-7aβ-methyl-1-oxo-3aα-H-hexa-hydro-indan-4α-yl)-propionsäure bzw. der entsprechenden δ-Lacton-Verbindung der zuvor angegebenen Struktur. Die neue Mikroorganismen-Defektmutante Chol 149-K 14 ist bei der Deutschen Sammlung von Mikroorganismen (DSM), Griesebach-Straße 8, D/3400, Göttingen, unter der Hinterlegungsnummer DSM 2065 hinterlegt.

Die Defektmutante DSM 2065 hat sich als besonders wirkungsvoll für den partiellen Abbau natürlicher Steroid-Verbindungen erwiesen. Geeignete Steroid-Ausgangsmaterialien für die Transformation sind natürliche Sterine wie Cholesterin, Sitosterin, Stigmasterin und/oder Ergosterin. Auch Abkömmlinge hiervon wie Cholestenon, Sitostenon, Stigmastenon und dergleichen sind geeignet. Das gleiche gilt für Gallensäuren, wie Cholsäure, Desoxycholsäure, Lithocholsäure und deren Gemische. Aber auch weitere Verbindungen mit der Steroidgrundstruktur sind geeignet, beispielsweise parzielle Abbauprodukte von natürlichen Sterinen, in denen der Substituent C 17 bereits partiell oder vollständig abgebaut worden ist.

Die technische Durchführung der Abbaureaktion erfolgt in grundsätzlich bekannter Weise. So kann beispielsweise die als Einsatzmaterial gewählte Steroid-Verbindung der Mikroorganismen Kultur während der Inkubationsperiode zugesetzt werden, oder sie kann dem Nährmedium vor der Inokulierung der Defektmutante beigegeben werden. Es kann eine Steroid-Verbindung oder auch eine Mischung von mehreren Steroid-Verbindungen eingesetzt werden. Vorzugsweise werden die Steroide der Kultur in Mengen von etwa 0,1-100 g/l zugesetzt. Die optimale Konzentration der zu transformierenden Steroid-Verbindung in der Züchtungsstufe kann durch einfache Vorversuche ermittelt werden. Im allgemeinen liegt die Konzentration der Steroid-Verbindung im Medium bevorzugt nicht über 50 g/l und häufig nicht über 30 g/l, jedoch werden Mengen über 1 g/l bevorzugt.

Es kann dabei weiterhin bevorzugt sein, das dem partiellen Abbau zu unterwerfende Substrat nicht auf einmal dem Reaktionsmedium zuzusetzen, sondern diese Zugabe nach und nach im Verlauf der Reaktion vorzunehmen. Vorzugsweise wird das Ausgangssubstrat in dieser Ausführungsform im wesentlichen kontinuierlich dem Reaktionsgemisch im Verlauf der Abbaureaktion zugegeben. Auf diese Weise kann die Ausbeute am gewünschten Abbauprodukt erhöht werden.

Die Kultur wird in einem Nährmedium gezüchtet, das als Kohlenstoffquelle entweder die zu transformierenden Steroid-Verbindungen oder aber auch noch zusätzliche metabolisierbare Kohlenstoffquellen, sowie die üblicherweise von diesen Mikroorganismen benötigten Nähr- und Wuchsstoffe enthält. Besonders günstig für das Wachstum der Mikroorganismen sind z. B. Paraffin, Glycerin, Carbonsäuren, Stärke, Dextrin, Saccharose, Glucose, Fruktose und zuckerhaltige Abfallstoffe. Geeignete Stickstoffquellen sind Ammoniumsalze, Nitrate, Pepton, Maisquellwasser, Sojamehl, Schlempe und Fischmehl. Weiterhin können Fermentationsbeschleuniger wie Hefeextrakt und Vitamine zugesetzt werden. Das Nährmedium enthält darüber hinaus üblicherweise anorganische Salze wie Natrium-, Kalium- oder Ammonium-phosphate oder Kalzium-, Magnesium-, Mangan- und/oder Eisensalze.

Die Emulgierung des Steroid-Ausgangsmaterials in dem Nährmedium erfolgt vorzugsweise mittels bekannter Emulgatoren z. B. mittels Fettsäure-Sorbitanester oder deren Ethylenoxidaddukten, Polyoxyethylen, Monolaurylether oder Fettsäureamidoalkylbetain.

Das verwendete Kulturmedium wird vor Beginn der Mikroorganismen-Anzucht zweckmäßigerweise durch Erhitzen sterisiliert. Nach dem Abkühlen und Beimpfen des Kulturmediums mit einer geeigneten Vorkultur der Defektmutante wird zwischen 25-55 °C inkubiert, vorzugsweise bei 27-30 °C. Der pH-Wert der Nährlösung liegt zwischen pH 4 und 8,5, vorzugsweise bei 6,8-8,0. Die Kultur wird durch Schütteln, Rühren oder Gaseinleiten mit Sauerstoff versorgt und bevorzugt bis zum vollständigen Abbau der Steroid-Verbindung bis zur gewünschten Stufe inkubiert.

Der Abbau der Steroid-Verbindung fordert je nach Substrat-Konzentration und den anderen Fermentationsbedingungen in der Regel 24-160 Stunden. Die Mitverwendung bestimmter Inhibitoren oder das Mikroorganismenwachstum einschränkender Wachstumshemmer ist nicht erforderlich.

Nach Abbruch der Fermentation wird überlicherweise die Zellmasse von der Kulturbrühe getrennt — beispielsweise durch Filtrieren — und das Verfahrensprodukt aus der Kulturbrühe isoliert. Als zweckmäßig hat es sich erwiesen, die Kulturbrühe — gegebenenfalls nach vorheriger Einengung — beispielsweise im Dünnschichtverdampfer — auf pH-Werte von 2,0 oder darunter anzusäuern. Als Verfahrensprodukt kann dan unmittelbar die Hexahydro-1-indanon-δ-Lactonverbindung gewonnen werden. Zum Ansäuern sind Mineralsäuren, aber auch organische Säuren geeignet, bevorzugt wird mit Schwefelsäure angesäuert.

Die gewünschtenfalls eingeengte und angesäuerte Kulturbrühe wird mit einem mit Wasser nicht mischbaren Lösungsmittel extrahiert. Geeignete Lösungsmittel sind beispielsweise Methyl-isopropylketon, Essigsäureester-hexanol-n-octanol, n-Hexan oder insbesondere halogenierte Kohlenwasserstoffverbindungen wie Chloroform oder Methylenchlorid. Nach Eindampfen der organischen Phase fällt das Reaktionsrohprodukt an, das in üblicher Weise beispielsweise durch Kristallisation, gereinigt werden kann.

Das erfindungsgemäß hergestellte Hexahydro-1-indanon-δ-Lacton bzw. die zugehörige freie substituierte Propionsäureverbindung sind wichtige Zwischenprodukte zur Herstellung pharmazeutisch aktiver Sterinverbindungen. Sie spielen insbesondere eine bedeutende Rolle bei der Synthese von 19-Norsteroiden.

## Beispiele

Der Stamm Chol 149/K14 (DSM 2065) wird im 75 l Fermenter (Füllvolumen 50 l) in folgendem Nährmedium bei 30 °C aerob angezüchtet :

0,50 Gewichtsprozent Glucose
0,5 Gewichtsprozent Hefeextrakt,
0,80 Gewichtsprozent Maisquellwasser,
1,50 Gewichtsprozent Maiskleber,
0,13 Gewichtsprozent $K_2HPO_4$,
0,09 Gewichtsprozent $(NH_4)$ 2x, $HPO_4$, pH 7,0.

Nach 24-stündiger Inkubation erfolgt die Zugabe von zunächst

0,1 Gewichtsprozent Cholesterin gemeinsam mit
0,05 Gewichtsprozent eines Emulgiermittels (DK-Ester F 50).

Nach weiteren 6 Stunden werden

0,5 Gewichtsprozent des Emulgiermittels plus
1,5 Gewichtsprozent Cholesterin plus
0,5 Gewichtsprozent Glucose

über einen Zeitraum von 36 Stunden zudosiert.

Nach 124 Stunden Fermentationszeit wird der Ansatz abgebrochen, die Zellmasse abfiltriert, die zellfreie Kulturbrühe im Dünnschichtverdampfer 3 : 1 aufkonzentriert, das Konzentrat mit wäßriger

Schwefelsäure auf pH 0,2 eingestellt und schließlich im Volumenverhältnis 1 : 1 mit Methylenchlorid extrahiert. Die Methylenchloridphase wird abgetrennt. Nach Eindampfen der organischen Phase fällt 3aα-H-4α-[3'-Propionsäure]-5α-hydroxy-7aβ-methylhexa-hydro-1-indanon-δ-Lacton in einer Ausbeute von ca. 63 mol % an.

**Patentanspruch**

Verwendung der Mikroorganismen-Defektmutante-Chol 149-K 14 (DSM 2065) zum mikrobiellen Abbau von Steroid-Verbindungen tierischen und pflanzlichen Ursprungs in einem wäßrigen Nährmedium unter aeroben Bedingungen zu 3aα-H-4α-[3'-propionsäure]-5α-Hydroxy-7aβ-methyl-hexahydro-1-indanon bzw. seinem δ-Lacton.

**Claim**

The use of the microorganism defect mutant chol 149-K 14 (DSM 2065) for the microbial degradation of steroid compounds of animal and vegetable origin in a aqueous nutrient medium under aerobic conditions to 3aα-H-4α-[3'-propionic acid]-5α-hydroxy-7aβ-methylhexahydro-1-indanone or its δ-lactone.

**Revendication**

Utilisation du microorganisme mutant par défaut-Chol 149-K 14 (DSM 2065) pour la dégradation microbienne de composés stéroïdes d'origine animale et végétale, dans un milieu nutritif aqueux, dans des conditions aérobies, en 3aα-H-4α-[acide 3'-propionique]-5α-hydroxy-7αβ-méthyl-hexahydro-1-indanone, ou en δ-lactone de ce composé.